Europäisches Patentamt

(19) European Patent Office

Office européen des brevets

(11) Veröffentlichungsnummer: **0 113 824**
**B1**

(12) **EUROPÄISCHE PATENTSCHRIFT**

(45) Veröffentlichungstag der Patentschrift:
16.09.87

(21) Anmeldenummer: 83111171.1

(22) Anmeldetag: 09.11.83

(51) Int. Cl.⁴: **D 01 F 2/28**, C 08 B 3/06,
C 08 B 3/08, A 61 L 15/00

(54) **Wasserunlösliche Fasern aus Celluloseacetat, Cellulosepropionat und Cellulosebutyrat mit einem extrem hohen Absorptionsvermögen für Wasser und physiologische Flüssigkeiten und Verfahren zu deren Herstellung.**

(30) Priorität: **15.12.82 DE 3246417**

(43) Veröffentlichungstag der Anmeldung:
**25.07.84 Patentblatt 84/30**

(45) Bekanntmachung des Hinweises auf die Patenterteilung:
**16.09.87 Patentblatt 87/38**

(84) Benannte Vertragsstaaten:
**AT BE CH DE FR GB IT LI LU NL SE**

(56) Entgegenhaltungen:
**DE - A - 3 227 267**
**GB - A - 527 565**
**US - A - 4 278 790**
**US - A - 4 289 130**

(73) Patentinhaber: **Akzo GmbH,**
**Postfach 10 01 49 Kasinostrasse 19-23,**
**D-5600 Wuppertal-1 (DE)**

(72) Erfinder: **Diamantoglou, Michael, Dr. Dipl.-Chem.,**
**Kolpingstrasse 4, D-8765 Erlenbach (DE)**
Erfinder: **Brandner, Alexander, Dr. Dipl.-Chem.,**
**Blockhausweg 4, A-4662 Steyrermühl (AT)**
Erfinder: **Meyer, Gerhard, Dr. Dipl.-Chem.,**
**Blumenstrasse 26, D-8753 Obernburg (DE)**

## Beschreibung

Die Erfindung betrifft wasserunlösliche Fasern aus Celluloseacetat, Cellulosepropionat und Cellulosebutyrat mit einem extrem hohen Absorptionsvermögen für Wasser und physiologische Flüssigkeiten und ein Verfahren zu ihrer Herstellung.

Es besteht nach wie vor ein Bedürfnis nach Faserartikeln mit verbesserter Saugfähigkeit in den Bereichen Hygiene, Medizin, Haushalt, Kleidung und Technik. Die immer schärferen physiologischen Anforderungen insbesondere in den Bereichen Hygiene und Medizin engen hierbei die Auswahlmöglichkeiten von hydrophilen Fasermodifikationen in nicht unerheblicher Weise ein.

Hydrophil modifizierte Viskosefasern sind unter dem Handelsnamen Viscosorb bekannt (vgl. Lenzinger Berichte, Heft 51 [1981], Seite 34ff). Wenn sie auch teilweise den Anforderungen des DAB und des EAB entsprechen, so erscheint ihr Wasserrückhaltevermögen noch verbesserungsbedürftig.

Celluloseacetate sind in Form von amorphen Produkten und Fasern seit langem bekannt. Acetatfasern werden mit einem Essigsäuregehalt von etwa 53–55% hergestellt, was einem Veresterungsgrad von 2,3 entspricht. Die bekannten Celluloseacetate sind bei einem Veresterungsgrad von 1,2 bis 3 wasserunlöslich, bei den niedrigeren Veresterungsgraden von 0,6 bis 0,9 sind sie dagegen wasserlöslich (vgl. Ullmann, Band 9 [1975], Seiten 233 und 238).

In der US-A 4 278 790 werden homogene Cellulose-Lösungen in LiCl und Dimethylacetamid beschrieben. Sie enthalten bis zu 3 Gew.% Cellulose und bis zu 8 Gew.% LiCl. Es wird darin festgestellt, dass Lösungen, die bis zu 8 bis 10 Gew.% Cellulose enthalten, zwar unter Druck hergestellt werden können, dass sie jedoch relativ viskos und unstabil sind. Aus diesem Grunde werden in den Beispielen der US-A 4 278 790 die an sich bekannten Umsetzungen der Cellulose zu mannigfaltigen Cellulosederivaten mit einem DS von <0.01 bis 3, z.B. Monocarbonsäurederivaten, mit den oben erwähnten niedrigen Cellulosekonzentrationen durchgeführt. Die Herstellung von wasserunlöslichen Fasern, die nur in engen DS-Bereichen ein extrem hohes Absorptionsvermögen für Wasser und physiologische Flüssigkeiten aufweisen, wird in der US-A 4 278 790 nicht offenbart.

Die vorliegende Erfindung stellt sich die Aufgabe, neue wasserunlösliche Fasern mit einem verbesserten Absorptionsvermögen für Wasser und physiologische Flüssigkeiten bereitzustellen, die darüber hinaus auch den gestiegenen physiologischen Anforderungen in den Bereichen Hygiene und Medizin genügen.

Gegenstand der Erfindung sind wasserunlösliche Fasern aus Celluloseacetat, Cellulosepropionat und Cellulosebutyrat mit einem extrem hohen Absorptionsvermögen für Wasser und physiologische Flüssigkeiten, die dadurch gekennzeichnet sind, dass sie

a) in der genannten Reihenfolge einen Veresterungsgrad von 0,1 bis 1,5, 0,1 bis 0,95 und 0,15 bis 0,8,

b) einen Durchschnittspolymerisationsgrad von 300 bis 700,

c) eine Faserfestigkeit im konditionierten Zustand von 6 bis 20 cN/tex,

d) eine Dehnung im konditionierten Zustand von 6 bis 20%,

e) eine Wasserrückhaltevermögen von 200 bis 750% und

f) eine Wasser-Saugkapazität von 8 bis 18 cm³/g Faser aufweisen.

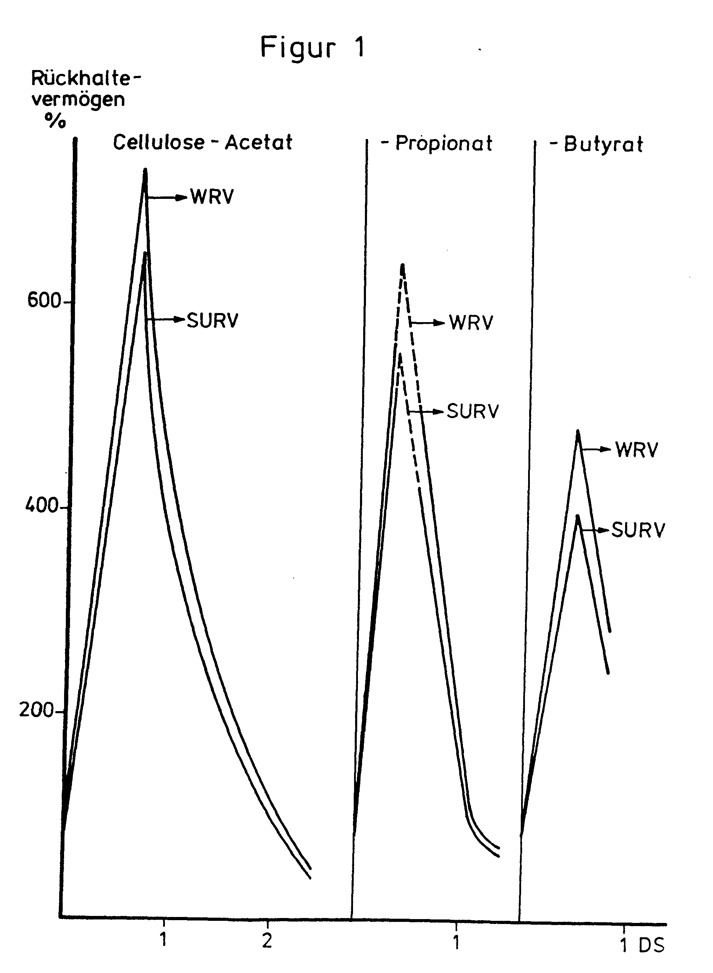

Es ist überraschend, dass die enorme Steigerung des Absorptionsvermögens für Wasser und physiologische Flüssigkeiten bei den erfindungsgemässen Fasern nur in sehr eng begrenzten Bereichen des Veresterungsgrades möglich ist, Dies sei anhand der Figur 1 demonstriert, die das Wasserrückhaltevermögen (WRV) und das Rückhaltevermögen von synthetischem Urin (SURV) der erfindungsgemässen Fasern in Abhängigkeit des Veresterungsgrades oder Substitutionsgrades aufzeigt. Das Wasserrückhaltevermögen nach DIN 53 814 ist ein Mass für das in den Einzelfasern nach ausgiebiger Immersion in Wasser und anschliessendem definierten Abschleudern festgehaltene Wasser. Gleiches gilt für das Rückhaltevermögen von synthetischem Urin, das nach der gleichen Vorschrift gemessen wurde.

Bei den erfindungsgemässen Celluloseacetatfasern steigen die WRV-Werte schon bei sehr niedrigen Veresterungsgraden steil an und erreichen bei einem Veresterungsgrad (DS) von 0,13 (entsprechendes gilt für die entsprechenden SURV-Werte) bereits den beachtlichen WRV-Wert von 200%. Das Wasserrückhaltevermögen durchläuft sodann bei einem Veresterungsgrad von 0,70 ein Maximum von 720%, um darauf bei weiter steigenden Veresterungsgraden kontinuierlich steil abzufallen. Die gleichen Gesetzmässigkeiten gelten auch für die erfindungsgemässen Cellulosepropionat- und Cellulosebutyratfasern mit der Massgabe, dass die erzielbaren WRV- und SURV-Maxima mit zunehmender Kettenlänge der Monocarbonsäure etwas niedriger werden und sich ebenso die obere Grenze des empfehlenswerten Veresterungsgradbereiches nach niedrigeren Werten hin verschiebt. Aus der Figur 1 ist fernerhin zu erkennen, dass die jeweiligen Differenzen aus den entsprechenden WRV- und SURV-Werten vergleichsweise klein sind, was die Anwendungsbreite der erfindungsgemässen Fasern in vorteilhafter Weise erweitert.

Die Kurven lassen erkennen, dass besonders hohe WRV-Werte und SURV-Werte erhalten werden, wenn der Veresterungsgrad der Celluloseacetatfasern in dem Veresterungsgradbereich von 0,5 bis 0,8, der Cellulosepropionatfasern in dem Veresterungsgradbereich von 0,25 bis 0,55 und der Cellulosebutyratfasern in dem Veresterungsgradbereich von 0,35 bis 0,55 liegt. Diese Werte gelten für den erfindungsgemässen Durchschnittspolymerisationsgrad der Fasern von 300 bis 700, der die oben schon angegebenen und für die spezifischen Anwendungszwecke mehr als zufriedenstellenden Faserfestigkeiten und Faserdehnungen positiv beeinflusst.

Demgemäss werden Fasern aus Celluloseacetat, Cellulosepropionat und Cellulosebutyrat bevorzugt, die

a) in der genannten Reihenfolge einen Veresterungsgrad von 0,5 bis 0,8, 0,25 bis 0,55 und 0,35 bis 0,55

b) eine Faserfestigkeit im konditionierten Zustand von 10 bis 16 cN/tex,

c) eine Dehnung im konditionierten Zustand von 8 bis 12% und

d) ein Wasserrückhaltevermögen von 400% bis 720% aufweisen.

Es ist hierbei überraschend, dass die erfindungsgemässen Celluloseacetatfasern in dem bevorzugten Veresterungsgradbereich von 0,5 bis 0,8 absolut wasserunlöslich sind. Wie oben bereits dargelegt wurde, sind die nach anderen Verfahren hergestellten Celluloseacetatfasern des Standes der Technik in diesem Bereich wasserlöslich, so dass sie für die hierin beabsichtigten Einsatzgebiete gar nicht in Betracht gezogen werden können.

Neben dem extrem hohen Absorptionsvermögen für Wasser und physiologische Flüssigkeiten weisen die erfindungsgemässen wasserunlöslichen Fasern auch eine gesteigerte Wasser-Saugkapazität auf. Diese wird im Demand-Wettability-Test (vgl. Bernard M. Lichstein, INDA, 2nd Annual Symposium on Nonwoven Product Development, March 5 & 6, 1974, Washington, D.L.) gemessen, der als sehr anwendungsbezogener Test die mittlere Sauggeschwindigkeit und die Saugkapazität eines Saugstoffes auch unter einem bestimmten Auflagedruck aufweist, wobei die Messflüssigkeit selbst keinen Druck auf die Probe ausübt. Da die erfindungsgemässen Fasern einen Faser-$p_H$-Wert von unter 7 besitzen und keine wasserlöslichen Anteile enthalten, erfüllen diese die Anforderungen des DAB und des EAB. Sie können also auch dort angewendet werden, wo zur Lösung des Absorptionsproblems unmittelbarer Faserkontakt zu offenen Wunden oder empfindlichen Schleimhäuten erforderlich ist, also in Form von Tampons, Wundtupfern, Absorptionsverbänden usw. Als weitere Anwendungszwecke seien zum Beispiel die Herstellung von Windeln, Wischtüchern, Dunstfiltern und Telefonkabelisolierungen genannt.

Die Erfindung betrifft ausserdem ein Verfahren zur Herstellung der in Rede stehenden wasserunlöslichen Celluloseacetat-, Cellulosepropionat- und Cellulosebutyratfasern. Dieses ist dadurch gekennzeichnet, dass man

a) bei einer Temperatur von 20 bis 80°C eine Lösung von aktivierter Cellulose in Dimethylacetamid oder 1-Methyl-2-pyrrolidon herstellt, die 5 bis 13 Gewichtsprozent aktivierte Cellulose mit einem Durchschnittspolymerisationsgrad von 400 bis 800 und 3 bis 10 Gewichtsprozent LiCl enthält,

b) die so gelöste Cellulose mit den entsprechenden Säureanhydriden in einem Molverhältnis von 1 : 0,5 bis 1 : 4 bei 20 bis 50°C in Gegenwart von an sich bekannten Veresterungskatalysatoren, bis zu den in den Ansprüchen 1 und 2 jeweils genannten Veresterungsgraden umsetzt und

c) die so erhaltenen Celluloseesterlösungen durch Nassspinnen in einem Koagulationsmittel verspinnt.

Die Herstellung der LiCl enthaltenden Lösungen von aktivierter Cellulose in Dimethylacetamid oder 1-Methyl-2-pyrrolidon ist nach der DE-A 3 027 033 bekannt. Diese Literaturstelle beschreibt mehrere Verfahrensvarianten zur Aktivierung der Cellulose und zur Herstellung der genannten Lösungen.

Bisher wurden jedoch Celluloseester des hier in Rede stehenden niedrigen Essigsäuregehaltes über die Triesterstufe durch Hydrolyse erhalten. Durch Zugabe von Wasser wurde der Überschuss an Anhydrid zerstört und durch Zugabe weiterer Schwefelsäure eine bestimmte Anzahl von Acetylgruppen wieder abgespalten. Der Verbrauch an Essigsäureanhydrid und Schwefelsäure war entsprechend hoch (Ullmann, Band 9 [1975], Seite 228, 230).

Demgegenüber ist das erfindungsgemässe Herstellungsverfahren dadurch gekennzeichnet, dass man die organischen Celluloselösungen nur bis zu den oben definierten niedrigen und eng begrenzten Veresterungsgraden umsetzt. Hierbei wird nicht nur eine Einsparung an Essigsäureanhydrid und Katalysatoren erzielt, sondern es werden überraschenderweise insbesondere Materialien einer gänzlich andersgearteten und bisher unbekannten Qualität erhalten. Es wurde oben schon darauf hingewiesen, dass z.B. die bevorzugten erfindungsgemässen Celluloseacetatfasern eines Veresterungsgrades 0,5–0,8 nach den Herstellungsmethoden des Standes der Technik wasserlöslich sind und demzufolge ganz andere physikalische Eigenschaften aufweisen. Aufgrund des andersgearteten Herstellungsverfahrens und der gezielten Auswahl eng begrenzter Bereiche eines niedrigen Veresterungsgrades besitzen die erfindungsgemässen Celluloseester im Vergleich zu denen des Standes der Technik offensichtlich eine unterschiedliche chemische Konstitution.

Als an sich bekannte Veresterungskatalysatoren eignen sich für die Veresterungsreaktion Säuren, wie Methansulfonsäure, Perchlorsäure, Ameisensäure und Schwefelsäure, oder Säurechloride, wie Acetylchlorid und Propionylchlorid. Diese sauren Veresterungskatalysatoren werden in Mengen von etwa 2 bis 10 Gewichtsprozent, bezogen auf die Säureanhydridmenge, eingesetzt.

Aber auch basische Veresterungskatalysatoren sind für diese in Rede stehenden Veresterungsreaktionen gut geeignet, zumal sie einem Abbau der Cellulose entgegenwirken. Als Beispiele solcher Verbindungen seien genannt: 4-N,N-Dimethylaminopyridin, Collidin, Pyridin und Triäthylamin. Derartige basische Veresterungskatalysatoren werden, bezogen auf das Säureanhydrid, in äquimolaren Mengen zugesetzt, um die bei der Reaktion freiwerdende Säure zu binden. Als Veresterungskatalysatoren eignen sich ebenfalls basische Salze von Monocarbonsäuren, wie Natriumacetat, Kaliumacetat, Natriumpropionat, Kaliumpropionat, Natriumbutyrat und Kaliumbutyrat. Im allgemeinen werden diese Salze, bezogen auf das Säureanhydrid, in Mengen von 6–20 Gewichtsprozent, vorzugsweise von 8–13 Gewichtsprozent, eingesetzt.

Besonders vorteilhafte Produkte erhält man, wenn man zur Herstellung der Celluloseesterfasern eine Lösung von aktivierter Cellulose in Dimethylacetamid herstellt, die 8 bis 12 Gewichtsprozent aktivierte Cellulose mit einem Durchschnittspolymerisationsgrad von 600 bis 800 enthält, bei einer Temperatur von 20° bis 30°C mit den entsprechenden Säureanhydriden umsetzt und das Reaktionsprodukt danach verspinnt. Bei diesen verwendeten niedrigen Reaktionstemperaturen kann ein Abbau der Cellulose weitgehend vermieden werden, was in Kombination mit den erhöhten Cellulosemolekulargewichten und Lösungskonzentrationen die mechanischen Eigenschaften der versponnenen Fasern positiv beeinflusst.

Die erfindungsgemässen Celluloseesterlösungen werden nach herkömmlichen Nassspinnverfahren und mit herkömmlichen derartigen Vorrichtungen versponnen. Bei Nassspinnen wird die entsprechend hergestellte Celluloseesterlösung durch Düsen mit feinen Bohrungen in ein geeignetes Koagulationsbad, beispielsweise ein bei 20° bis 60°C gehaltenes Wasserbad, ausgepresst. Zu anderen brauchbaren Koagulationsmitteln gehören Mischungen von Wasser und wasserlöslichen organischen Lösungsmitteln, wie Alkohole, Ketone, Äther oder das zur Lösung der Cellulose verwendete Lösungsmittel. Die Entwicklung von maximalen Fasereigenschaften kann unterstützt werden, indem die zu einem Spinnkabel vereinigten Fäden eine Reihe wässriger oder organischer Lösungsmittel und gegebenenfalls anorganischer Salze enthaltende Waschbäder durchlaufen, um Reste der verwendeten Lösungsmittel und LiCl zu entfernen. Gleichzeitig kann eine Verstreckung mit der Nachbehandlung verbunden werden, die die Einstellung der jeweils gewünschten mechanischen Fasereigenschaften gestattet. Das Verstreckungsverhältnis kann hierbei von 1:1 bis 3:1 variiert werden.

Die Erfindung wird anhand der folgenden Beispiele näher erläutert:

Beispiel 1

In einem 1 l Dreihalskolben werden 16,2 g (0,1 Mol) Cellulose (DP: 700, gemessen im Lösungsmittel Cuen) in 278,4 g (3,2 Mol) technischem Dimethylacetamid suspendiert und bei 155°C 30 Minuten lang aktiviert. Nach dem Abkühlen auf 100°C werden 29 g (0,68 Mol) LiCl zugesetzt, dabei steigt die Temperatur um 5–10°C an; anschliessend wird auf Raumtemperatur (RT stets 20–25°C) abgekühlt. Nach 2–3 Stunden Rühren bei Raumtemperatur wird eine gelartige Celluloselösung erhalten. Es wird über Nacht weiter gerührt. Dabei entsteht eine klare viskose Celluloselösung, die mit einem Gemisch von 12,24 g (0,12 Mol) Essigsäureanhydrid und 1,18 g (0,015 Mol) Acetylchlorid 20 Stunden bei Raumtemperatur acetyliert wird. Das Reaktionsgemisch wird entlüftet und durch eine Viskosespinndüse (36/90) in ein Fällbad aus Wasser/Alkohol (30/70) versponnen, gewaschen und getrocknet.

Die auf diese Weise erhaltenen Celluloseacetatfasern weisen folgende Eigenschaften auf:

| | | |
|---|---|---|
| Veresterungsgrad (DS): | 0,60 | |
| Polymerisationsgrad (DP): | 570 | |
| Faserfestigkeit kond.: | 12,4 | cN/tex |
| Faserdehnung kond.: | 8,9 | % |
| Wasserrückhaltevermögen (WRV): | 640 | % |
| synth. Urinrückhaltevermögen (SURV): | 560 | % |

Beispiele 2–20

Die in den nachfolgenden Tabellen 1–6 aufgeführten Celluloseacetate wurden im Prinzip nach dem gleichen Verfahren hergestellt bzw. versponnen, wie in Beispiel 1 angegeben.

a) Die in Tabelle 1 für die Herstellung von Celluloseacetatfasern aufgeführten Beispiele 2–7 zeigen den Einfluss variabler Essigsäureanhydridmengen auf. Hierbei werden Art und Menge des Katalysators, die Reaktionstemperatur und die Reaktionszeit konstant gehalten.

b) In Tabelle 2 wird anhand der Beispiele 3 und 8–12 die Abhängigkeit des Veresterungsgrades von verschiedenen sauren Katalysatorarten aufgezeigt. Hierbei wurde die Reaktionstemperatur, die Reaktionszeit, die Katalysatormenge und das Molverhältnis von Cellulose zu Säureanhydrid konstant gehalten.

c) In Tabelle 3 wird anhand der Beispiele 3 und 13–14 auf der Basis des Katalysators Acetylchlorid der Einfluss ansteigender Katalysatorkonzentrationen aufgezeigt. In diesen Versuchen wurde mit den konstanten Parametern Reaktionstemperatur, Reaktionszeit und Molverhältnis Cellulose zu Säureanhydrid gearbeitet.

d) Die in Tabelle 4 aufgeführten Beispiele 3 und 15–16 zeigen den Einfluss variabler Reaktionszeiten auf. Als Katalysator wurde 2 Gew.% Acetylchlorid verwendet und auch die Reaktionstemperatur und das Molverhältnis von Cellulose zu Säureanhydrid wurden konstant gehalten.

e) Tabelle 5 zeigt den Einfluss der Variable Reaktionstemperatur. In den Beispielen 15 und 17–18 wurde Art und Menge des Katalysators Acetylchlorid, die Reaktionszeit und das Molverhältnis Cellulose zu Säureanhydrid konstant gehalten.

f) Dass der Veresterungsgrad auch erheblich von der Reinheit des verwendeten Lösungsmittels abhängig ist, wird in Tabelle 6 anhand von Dimethylacetamid demonstriert. In den Beispielen 3, 13, 19–20 wurde mit den konstanten Parametern Reaktionstemperatur, Reaktionszeit, Katalysator und Molverhältnis Cellulose zu Säureanhydrid gearbeitet.

Tabelle 1 (Celluloseacetat)
Veresterung von Cellulose mit wechselnden Essigsäureanhydridmengen

Reaktionstemperatur: RT; Reaktionszeit: 20 h
Katalysator:          2 Gew.% Acetylchlorid, bezogen auf Säureanhydrid

| Beispiel | Molverhältnis | | DS | DP | WRV (%) | SURV (%) | Faserfestigkeit kond. (cN/tex) | Faserdehnung kond. (%) |
|---|---|---|---|---|---|---|---|---|
| | Cellulose | Säureanhydrid | | | | | | |
| 2 | 1 | 1,0 | 0,13 | 650 | 270 | 235 | 19,6 | 12,0 |
| 3 | 1 | 1,5 | 0,23 | 610 | 310 | 270 | 16,3 | 11,4 |
| 4 | 1 | 2,0 | 0,57 | 560 | 635 | 540 | 12,1 | 8,5 |
| 5 | 1 | 2,5 | 0,82 | 520 | 545 | 460 | 12,7 | 9,7 |
| 6 | 1 | 3,0 | 0,89 | 525 | 470 | 395 | 13,5 | 10,6 |
| 7 | 1 | 4,0 | 1,50 | 510 | 240 | 205 | 14,8 | 11,3 |

Tabelle 2
Abhängigkeit des Veresterungsgrades von der Katalysatorart

Reaktionstemperatur: RT; Reaktionszeit: 20 h
Molverhältnis:          Cellulose:
                        Säureanhydrid = 1:1,5

| Beispiel | Katalysator, bezogen auf Säureanhydrid | | DS |
|---|---|---|---|
| 3 | $CH_3COCl$ | 2 Gew.% | 0,23 |
| 8 | $CH_3SO_3H$ | 2 Gew.% | 0,38 |
| 9 | $HClO_4$ | 2 Gew.% | 0,24 |
| 10 | $H_2SO_4$ | 2 Gew.% | 0,13 |
| 11 | HCOOH | 2 Gew.% | 0,15 |
| 12 | ohne | | 0,11 |

Tabelle 3 (Celluloseacetat)
Abhängigkeit des Veresterungsgrades von den Katalysatormengen

Reaktionstemperatur: RT; Reaktionszeit: 20 h
Molverhältnis:          Cellulose:
                        Säureanhydrid = 1:1,5

| Beispiel | Katalysator, bezogen auf Säureanhydrid | | DS |
|---|---|---|---|
| 3 | Acetylchlorid | 2 Gew.% | 0,23 |
| 13 | Acetylchlorid | 5 Gew.% | 0,58 |
| 14 | Acetylchlorid | 10 Gew.% | 0,78 |

Tabelle 4 (Celluloseacetat)
Abhängigkeit des Veresterungsgrades von der Reaktionszeit

Molverhältnis:          Celluose: Säureanhydrid
                        = 1:1,5
Katalysator:            Acetylchlorid; 2 Gew.%,
                        bezogen auf Säureanhydrid
Reaktionstemperatur: RT

| Beispiel | Reaktionszeit | DS |
|---|---|---|
| 3 | 20 | 0,23 |
| 15 | 6 | 0,20 |
| 16 | 48 | 0,45 |

Tabelle 5 (Celluloseacetat)
Abhängigkeit des Veresterungsgrades von der Reaktionstemperatur

Molverhältnis: Cellulose: Säureanhydrid = 1:1,5
Katalysator:    Acetylchlorid 2 Gew.%, bezogen
                auf Säureanhydrid
Reaktionszeit: 6 Stunden

| Beispiel | Reaktionstemperatur (°C) | DS |
|---|---|---|
| 15 | RT | 0,20 |
| 17 | 40–50 | 0,37 |
| 18 | 60–70 | 0,48 |

Tabelle 6 (Celluloseacetat)
Abhängigkeit des Veresterungsgrades von der Reinheit des Lösungsmittels

Reaktionstemperatur: RT, Reaktionszeit: 20 h
Molverhältnis:          Cellulose:
                        Säureanhydrid = 1:1,5

| Beispiel | Katalysator, bezogen auf Säureanhydrid | | DS | Bemerkung |
|---|---|---|---|---|
| 3 | $CH_3COCl$ | 2 Gew.% | 0,23 | techn. DMAc |
| 13 | $CH_3COCl$ | 5 Gew.% | 0,58 | techn. DMAc |
| 19 | $CH_3COCl$ | 5 Gew.% | 0,80 | p.a. DMAc |
| 20 | $CH_3COCl$ | 5 Gew.% | 0,98 | p.a. DMAc |

Beispiele 21–28
Auf der Grundlage der Arbeitsweise von Beispiel 1 und den Reaktionsbedingungen der Tabelle 7 wurden in den Beispielen 21–28 Celluloseacetate hergestellt. Hierbei wurden verschiedene basische Katalysatoren und unterschiedliche Molverhältnisse von Cellulose zu Säureanhydrid angewandt. Die Reaktionstemperatur und die Reaktionszeit wurden konstant gehalten. Die erhaltenen Celluloseacetate lassen sich wie in Beispiel 1 leicht verspinnen.

Tabelle 7 (Celluloseacetat)
Veresterung von Cellulose mit Essigsäureanhydrid in Gegenwart eines tert. Amines

Reaktionstemperatur: RT; Reaktionszeit: 20 h;

| Beispiel | Molverhältnis | | tert. | Amin | DS | DP |
|---|---|---|---|---|---|---|
| | Cellulose | Säureanhydrid | | | | |
| 21 | 1 | 0,25 | 0,25 | Mol 4-N,N-Dimethylaminopyridin | 0,19 | 645 |
| 22 | 1 | 0,50 | 0,50 | Mol 4-N,N-Dimethylaminopyridin | 0,42 | 625 |
| 23 | 1 | 1,00 | 1,00 | Mol 4-N,N-Dimethylaminopyridin | 0,69 | 580 |
| 24 | 1 | 1,50 | 1,50 | Mol 4-N,N-Dimethylaminopyridin | 1,11 | 545 |
| 25 | 1 | 2,00 | 2,00 | Mol 4-N,N-Dimethylaminopyridin | 1,52 | – |
| 26 | 1 | 1,50 | 1,50 | Mol Collidin | 0,54 | 317 |
| 27 | 1 | 1,50 | 1,50 | Mol Pyridin | 0,87 | 316 |
| 28 | 1 | 1,50 | 1,50 | Mol Triäthylamin | 1,20 | – |

Beispiele 29–33
Auf der Grundlage der Arbeitsweise von Beispiel 1 und den Reaktionsbedingungen der Tabelle 8 werden in den Beispielen 29–33 Cellulosepropionate hergestellt und zu Fasern versponnen.

Als Veresterungskatalysator fungierte eine konstante Menge Propionylchlorid. Während das Molverhältnis von Cellulose zu Säureanhydrid variiert wurde, wurden die Reaktionstemperatur und die Reaktionszeit konstant gehalten.

Tabelle 8 (Cellulosepropionat)
Veresterung von Cellulose mit wechselnden Propionsäureanhydridmengen

Reaktionstemperatur: RT; Reaktionszeit: 20 h
Katalysator: 2 Gew.% Propionylchlorid bezogen auf Säureanhydrid

| Beispiel | Molverhältnis | | DS | WRV (%) | SURV (%) | Faserfestigkeit kond. (cN/tex) | Faserdehnung kond. (%) |
|---|---|---|---|---|---|---|---|
| | Cellulose | Säureanhydrid | | | | | |
| 29 | 1 | 1,00 | 0,26 | 390 | 355 | 12,6 | 12,1 |
| 30 | 1 | 1,50 | 0,32 | 570 | 520 | 10,5 | 8,3 |
| 31 | 1 | 2,00 | 0,56 | 505 | 420 | – | – |
| 32 | 1 | 3,00 | 1,10 | 110 | 100 | 13,5 | 9,8 |
| 33 | 1 | 4,00 | 1,37 | 75 | 70 | 11,1 | 10,3 |

Beispiele 30–36
Die Beispiele 30–36 zeigen auf der Grundlage der Arbeitsweise von Beispiel 1 die bei der Herstellung von Cellulosepropionaten bestehende Abhängigkeit des Veresterungsgrades von der Art und Menge von zwei sauren Veresterungskatalysatoren auf. Die Reaktionstemperatur, die Reaktionszeit und das Molverhältnis von Cellulose zu Säureanhydrid wurden hierbei konstant gehalten.

Tabelle 9 (Cellulosepropionat)
  Abhängigkeit des Veresterungsgrades von Art
und Menge des Katalysators

Reaktionstemperatur: RT; Reaktionszeit: 20 h;
Molverhältnis:          Cellulose:
                        Säureanhydrid = 1:1,5

| Beispiel | Katalysator, bezogen auf Säureanhydrid | | DS |
|---|---|---|---|
| 30 | $CH_3CH_2COCl$ | 2 Gew.% | 0,32 |
| 34 | $CH_3CH_2COCl$ | 5 Gew.% | 0,73 |
| 35 | $CH_3SO_3H$ | 2 Gew.% | 0,55 |
| 36 | $CH_3SO_3H$ | 5 Gew.% | 0,76 |

**Beispiele 37–40**

Auf der Grundlage der Arbeitsweise von Beispiel 1 und den Reaktionsbedingungen der Tabelle 10 werden in den Beispielen 37–40 Cellulosebutyrate hergestellt und zu Fasern versponnen. Als Veresterungskatalysator wurde eine konstante Menge Butyrylchlorid eingesetzt. Während das Molverhältnis von Cellulose zu Säureanhydrid variiert wurde, wurde die Reaktionstemperatur und die Reaktionszeit konstant gehalten.

Tabelle 10 (Cellulosebutyrat)
  Veresterung von Cellulose mit wechselnden Buttersäureanhydridmengen

Reaktionstemperatur: RT; Reaktionszeit: 20 h;
Katalysator:          2 Gew.% Butyrylchlorid, bezogen auf Säureanhydrid

| Beispiel | Molverhältnis | | DS | WRV (%) | SURV (%) | Faserfestigkeit kond. (cN/tex) | Faserdehnung kond. (%) |
|---|---|---|---|---|---|---|---|
| | Cellulose | Säureanhydrid | | | | | |
| 37 | 1 | 1,00 | 0,32 | 275 | 245 | 12,4 | 9 |
| 38 | 1 | 1,25 | 0,44 | 475 | 390 | – | – |
| 39 | 1 | 1,50 | 0,50 | 460 | 385 | – | – |
| 40 | 1 | 1,75 | 0,80 | 285 | 245 | – | – |

**Beispiel 41**

16,2 g (0,1 Mol) Cellulose werden in 278,4 g (3,2 Mol) technischem Dimethylacetamid suspendiert und 30 Minuten bei 155°C aktiviert. Nach dem Abkühlen auf 100°C werden 29 g (0,68 Mol) LiCl zugesetzt. Anschliessend wird auf Raumtemperatur abgekühlt und über Nacht gerührt. Zur klaren, viskosen Lösung werden 1 g (0,01 Mol) Kaliumacetat (Katalysator) und 12,24 g (0,12 Mol) Essigsäureanhydrid zugesetzt. Zur Vervollständigung der Reaktion wird 6 Stunden bei 80°C weitergeführt, die erhaltene Celluloseacetatlösung entlüftet und, wie in Beispiel 1 beschrieben, zu Fasern versponnen.

Die so hergestellten Celluloseacetatfasern zeigen folgende Eigenschaften:

| | | |
|---|---|---|
| Veresterungsgrad: | 0,70 | |
| Polymerisationsgrad: | 650 | |
| Faserfestigkeit kond.: | 12,9 | cN/tex |
| Faserdehnung kond.: | 9,3 | |
| Wasserrückhaltevermögen: | 720 | % |
| synth. Urinrückhaltevermögen: | 650 | % |

**Patentansprüche**

1. Wasserunlösliche Fasern aus Celluloseacetat, Cellulosepropionat und Cellulosebutyrat mit einem extrem hohen Absorptionsvermögen für Wasser und physiologische Flüssigkeiten, dadurch gekennzeichnet, dass sie

  a) in der genannten Reihenfolge einen Veresterungsgrad von 0,1 bis 1,5, 0,1 bis 0,95 und 0,15 bis 0,8,

  b) einen Durchschnittspolymerisationsgrad von 300 bis 700,

  c) eine Faserfestigkeit im konditionierten Zustand von 6 bis 20 cN/tex,

  d) eine Dehnung im konditionierten Zustand von 6 bis 20%,

  e) ein Wasserrückhaltevermögen von 200 bis 750% und

  f) eine Wasser-Saugkapazität von 8 bis 18 cm³/g Faser aufweisen.

2. Fasern aus Celluloseacetat, Cellulosepropionat und Cellulosebutyrat nach Anspruch 1, dadurch gekennzeichnet, dass sie

  a) in der genannten Reihenfolge einen Veresterungsgrad von 0,5 bis 0,8, 0,25 bis 0,55 und 0,35 bis 0,55

b) eine Faserfestigkeit im konditionierten Zustand von 10 bis 16 cN/tex,

c) eine Dehnung im konditionierten Zustand von 8 bis 12% und

d) ein Wasserrückhaltevermögen von 400% bis 720% aufweisen.

3. Verfahren zur Herstellung der Celluloseacetat-, Cellulosepropionat- und Cellulosebutyratfasern gemäss den Ansprüchen 1 und 2, dadurch gekennzeichnet, dass man

a) bei einer Temperatur von 20 bis 80°C eine Lösung von aktivierter Cellulose in Dimethylacetamid oder 1-Methyl-2-pyrrolidon herstellt, die 5 bis 13 Gewichtsprozent aktivierte Cellulose mit einem Durchschnittspolymerisationsgrad von 400 bis 800 und 3 bis 10 Gewichtsprozent LiCl enthält,

b) die so gelöste Cellulose mit den entsprechenden Säureanhydriden in einem Molverhältnis von 1:0,5 bis 1:4 bei 20 bis 50°C in Gegenwart von an sich bekannten Veresterungskatalysatoren, bis zu den in den Ansprüchen 1 und 2 jeweils genannten Veresterungsgraden umsetzt und

c) die so erhaltenen Celluloseesterlösungen durch Nassspinnen in einem Koagulationsmittel verspinnt.

4. Verfahren nach Anspruch 3, dadurch gekennzeichnet, dass man zur Herstellung der Celluloseesterfasern eine Lösung von aktivierter Cellulose in Dimethylacetamid herstellt, die 8 bis 12 Gewichtsprozent aktivierte Cellulose mit einem Durchschnittspolymerisationsgrad von 600 bis 800 enthält, bei einer Temperatur von 20° bis 30°C mit den entsprechenden Säureanhydriden umsetzt und das Reaktionsprodukt danach verspinnt.

## Claims

1. Water-insoluble fibers of cellulose acetate, cellulose propionate and cellulose butyrate having an extremely high absorption power for water and physiological liquids, characterized in that they have

a) a degree of esterification of from 0.1 to 1.5, from 0.1 to 0.95 and from 0.15 to 0.8 in the order indicated,

b) an average degree of polymerization of from 300 to 700,

c) a fiber strength in the conditioned state of from 6 to 20 cN/tex,

d) an elongation in the conditioned state of from 6 to 20%,

e) a water retention capacity of from 200 to 750% and

f) a water uptake capacity of from 8 to 18 cm³/g fiber.

2. Fibers of cellulose acetate, cellulose propionate and cellulose butyrate as claimed in Claim 1, characterized in that they have

a) a degree of esterification of from 0.5 to 0.8, from 0.25 to 0.55 and from 0.35 to 0.55 in the order indicated,

b) a fiber strength in the conditioned state of from 10 to 16 cN/tex,

c) an elongation in the conditioned state of from 8 to 12% and

d) a water retention capacity of from 400% to 720%.

3. A process for the production of the cellulose acetate, cellulose propionate and cellulose butyrate fibers claimed in Claims 1 and 2, characterized in that

a) a solution of activated cellulose in dimethyl acetamide or 1-methyl-2-pyrrolidone containing from 5 to 13% by weight activated cellulose having an average degree of polymerization of from 400 to 800 and from 3 to 10% by weight LiCl is prepared at a temperature of from 20 to 80°C,

b) the cellulose thus dissolved is reacted with the corresponding acid anhydrides in a molar ratio of from 1:0.5 to 1:4 at 20 to 50°C in the presence of esterification catalysts known per se to the degrees of esterification mentioned in Claims 1 and 2 and

c) the cellulose ester solutions thus obtained are spun by wet-spinning in a coagulating agent.

4. A process as claimed in Claim 3, characterized in that, to produce the cellulose ester fibers, a solution of activated cellulose in dimethyl acetamide containing from 8 to 12% by weight activated cellulose having an average degree of polymerization of from 600 to 800 is prepared, reacted with the corresponding acid anhydrides at a temperature of from 20 to 30°C and the reaction product subsequently spun.

## Revendications

1. Fibres, insolubles dans l'eau, d'acétate de cellulose, de propionate de cellulose et de butyrate de cellulose, ayant un pouvoir d'absorption extrêmement élevé pour l'eau et les liquides physiologiques, caractérisées en ce qu'elles présentent

a) dans l'ordre indiqué, un degré d'estérification de 0,1 à 1,5, de 0,1 à 0,95 et de 0,15 à 0,8;

b) un degré moyen de polymérisation de 300 à 700;

c) une résistance des fibres à l'état conditionné de 6 à 20 cN/tex;

d) un allongement à l'état conditionné de 6 à 20%;

e) un pouvoir de rétention de l'eau de 200 à 750%; et

f) une capacité d'inhibition d'eau de 8 à 18 cm³/g de fibres.

2. Fibres d'acétate de cellulose, de propionate de cellulose et de butyrate de cellulose selon la revendication 1, caractérisée en ce qu'elles présentent

a) dans l'ordre indiqué, un degré d'estérification de 0,5 à 0,8, de 0,25 à 0,55 et de 0,35 à 0,55;

b) une résistance des fibres à l'état conditionné de 10 à 16 cN/tex;

c) un allongement à l'état conditionné de 8 à 12% et

d) un pouvoir de rétention d'eau de 400% à 720%.

3. Procédé de préparation des fibres d'acétate de cellulose, de propionate de cellulose et de butyrate de cellulose selon les revendications 1 et 2, caractérisé en ce que

a) on prépare, à une température de 20 à 80°C, une solution de cellulose activée dans du diméthylacétamide ou de la 1-méthyl-2-pyrrolidone, qui contient 5 à 13% en poids de cellulose activée et ayant un degré moyen de polymérisation de 400 à 800, ainsi que 3 à 10% en poids de LiCl;

b) on fait réagir la cellulose ainsi dissoute avec les anhydrides d'acide appropriés, dans un rapport molaire de 1:0,5 à 1:4, entre 20 et 50°C, en présence de catalyseurs d'estérification connus en soi, jusqu'à atteindre les degrés d'estérification indiqués respectivement dans les revendications 1 et 2; et

c) on file les solutions d'ester cellulosique ainsi obtenues par filature au mouillé dans un agent de coagulation.

4. Procédé selon la revendication 3, caractérisé en ce qu'on prépare, pour la préparation des fibres de cellulose, une solution de cellulose activée dans du diméthylacétamide, qui contient 8 à 12% en poids de cellulose activée et ayant un degré moyen de polymérisation de 600 à 800, en ce qu'on la fait réagir à une température de 20 à 30°C, avec les anhydrides d'acide appropriés, et en ce qu'on file ensuite le produit de la réaction.

**0 113 824**

# Figur 1